# EUROPEAN PATENT APPLICATION

(11) **EP 3 789 765 A1**
(43) Date of publication of application: **10.03.2021**
(21) Application number: 19196131.7
(22) Date of filing: 09.09.2019
(51) Int. Cl.: G01N 33/50, A01N 1/02, G01J 3/00

(54) **A METHOD FOR EVALUATING DAMAGE OF SOLID TISSUE**

(71) Applicant: ETH Zürich, 8092 Zürich (CH); Universität Zürich, 8006 Zürich (CH)
(72) Inventor: CLAVIEN, Pierre-Alain, 8802 Kilchberg (CH); DUTKOWSKI, Philipp, 78337 Öhningen (DE); HEFTI, Max, 8057 Zürich (CH); SCHULER, Martin, 8048 Zürich (CH); BECKER, Dustin, 8008 Zürich (CH); RUDOLF VON ROHR, Philipp, 4056 Basel (CH)
(74) Representative: Maikowski & Ninnemann Patentanwälte Partnerschaft mbB

(57) **Abstract**

The invention relates to a method for evaluating damage of an organ tissue, in particular ischemic damage/injury of an organ tissue, with the steps of measuring the concentration of at least one marker molecule in the perfusate of the organ tissue, wherein the measured concentration of the at least one marker molecule in the perfusate is used in at least one computer based prediction algorithm for generating at least one success score, wherein the success score has been previously defined based on at least one parameter value of at least one pre-defined parameter; wherein the at least one parameter value is determined after a transplantation of the organ tissue; and wherein based on the at least one success score at least one signal and/or at least one set of data is generated for facilitating the decision, if the organ tissue is suitable for transplantation or not.

## Description

The invention relates to a method for evaluating damage of an organ tissue, in particular ischemic damage/injury of an organ tissue, and a spectroscopic analysis unit for use in such a method.

### Description

Since the early days of liver transplantation (LT), graft quality has always been considered as key confounder for outcome. However, half a decade later, the assessment of liver quality and the decision to accept or decline a graft prior to transplantation still relies frequently on "gut feeling", in combination with donor demographics and past medical history.

*Ex situ* machine perfusion is a method, which is gaining increasing interest to potentially repair injured organs and to assess organ function. Furthermore, machine perfusion before implantation may offer an objective evaluation of the metabolic status of an organ.

The machine perfusion technology is used to preserve human organs (e.g. ex vivo) by perfusing the organ/tissue with perfusate for a certain time period. Depending on the perfusion temperature (hypotherm 0-12°C, subnormothermic 12-35°C and normothermic 35-37°C) and the used organ, the perfusate can be whole blood, a physiological saline solution, a perfusate based on an artificial oxygen carrier, modified blood (e.g. leukocyte depleted) or a tailor-made solution.

The clinical application of machine perfusion for human organs can be in the patient (in vivo, e.g. normothermic regional perfusion (NRP)) or outside of the patient (ex vivo). In case of hypothermic perfusion (e.g. at 4°C), the perfusion can be executed with or without an actively oxygenating device (oxygenator) being part of the perfusion loop (tube set). Therefore, the perfusion technology under hypothermic conditions can be oxygenated or non-oxygenated. With increasing perfusion temperature towards normothermic conditions, an oxygenator has to be part of the perfusion loop and also at least one oxygen carrier is required in the perfusate (e.g. red blood cells). The machine perfusion technology can generally be applied to all human organs (e.g. liver) and human tissue.

In 2012, a novel machine perfusion approach, hypothermic oxygenated perfusion (HOPE), was introduced into routine clinical practice to improve human liver grafts donated after circulatory death (DCD) prior to implantation.

Only a few parameters for metabolic and cellular damage have been quantified in machine liver perfusates, and the most prominent include perfusate lactate, transaminases, pH, bile production and bile quality. In contrast, two recent studies showed the predictive value of a more in depth analysis of cold flush outs at the end of cold storage preservation through metabolomics and glyconomics. Those findings point to a superior clinical relevance of specific metabolic pathways in the liver, compared to the quantification of released cytosolic compounds.

However, in the field of liver transplantation, no studies exist on reliable prediction of graft function during machine perfusion.

Thus, it is an object of the present invention to identify and test the predictive value of perfusate markers of organ tissues analyzed during machine perfusion, in particular HOPE perfusion, on the function of the organ tissue after transplantation, for example the function of the liver after liver implantation.

This object is solved by providing a method for evaluating damage of an organ tissue, in particular ischemic damage/injury of an organ tissue, and a spectroscopic analysis unit for use in such a method.

According to the present invention, a method for evaluating damage / injury of an organ tissue, in particular ischemic damage/injury of an organ tissue, is provided that comprises the following steps:
- measuring the concentration of at least one marker molecule (as a predictive target molecule) in the perfusate of the organ tissue (i.e. any fluid flowing through a tissue or organ),
- wherein the measured concentration of the at least one marker molecule in the perfusate is used in at least one computer based prediction algorithm for generating at least one success score,
- wherein the success score has been previously defined based on at least one parameter value of at least one pre-defined parameter;
- wherein the at least one parameter value is determined after a transplantation of the organ tissue; and
- wherein based on the at least one success score at least one signal and/or at least one set of data is generated for facilitating the decision, if the organ tissue is suitable for transplantation or not.

Thus, a method is provided that allows for predicting the prospects or chances of success of an organ transplantation prior organ transplantation. With the present method, it is possible to assess the quality of an organ tissue, since the determined success score reflects the degree of organ tissue damage. The signal or data set provided by the present method allows for a yes / no -statement, if an organ transplantation may be successful or not.

In an embodiment of the present method the at least one success core corresponds to the concentration of the at least one marker molecule (predictive target molecule) in the perfusate, wherein a previously determined threshold value of the at least one marker molecule is used for generating the at least one signal and/or at least one set of data for facilitating the decision of an organ issue transplantation prior to the organ tissue transplantation. For example, in case the concentration of the marker molecule is below a certain threshold, then the organ may be used for transplantation, but when the concentration of the marker molecule is above a certain threshold, then the organ may not be used for transplantation.

The present method uses a marker molecule (such as FMN) as indicator for damage, amongst others, to characterize damage in tissue and organs. For example, the amount of FMN is used as an indicator for ischemic tissue damage and/or reperfusion injury. In particular, the amount of FMN present in the blood after ischemic heart injury correlates positively with the degree of injury of the ischemic tissue. Therefore, a high amount of FMN reveals that there is more ischemic damage compared to when the amount of FMN is low.

Thus, the concentration of the at least one marker molecule in the perfusate can be used as a predictor for the quality of the organ tissue prior transplantation of the organ tissue and to group the organ tissue into risk groups.

In a further embodiment of the present method, the computer based prediction algorithm is a regression algorithm or classification algorithm, wherein the pre-defined parameters used by the least one prediction algorithm are pre-transplanted information and post-transplant parameters. The prediction algorithm uses information stored in a database, wherein the database comprises pre-transplant parameters and post-transplant parameters.

The computer model encompasses a corresponding set of prediction algorithms, each of which may have been learned on a unique pool of information of previously available pre-transplant information along with a set of the corresponding post-transplant parameter of interest of previous transplantations.

The computer model further combines the set of predicted post-transplant parameters and map those into a success score, preferably by weighing each parameter to emphasize certain post-transplant parameters more than others, and said success score is used to facilitate the decision process whether to transplant tissue or not.

In a most preferred embodiment of the present method, machine learning and artificial intelligence is applied for characterizing the condition of the organ tissue.

In the present method a number of clinical parameters can be used as pre-defined parameters in the prediction algorithm. The available information used to decide whether solid organ tissue is transplanted into a recipient, or to predict any other post-transplant parameter, is comprised of the continuous and/or point measurements of any or a combination of marker molecules (or predictive target molecules), in particular a spectroscopic measurement as will be described in detail below.

The at least one marker molecule (predictive target molecule) may be selected from a group comprising FMN, FAD, NADH, Alanine Aminotransferase (ALT), Aspartate Aminotransferase (AST), Glucose, lactate, wherein FMN, NADH and FAD are the most preferred marker molecules.

This information is passed to the prediction algorithm and is henceforth termed *pre-transplant information.* In a further embodiment of the present invention, the pre-transplant information includes also information about the donor of the solid-organ tissue, e.g. sex, age, cause of death, ethnicity, medical records, medical condition, height, body mass index, ischemia time of the solid organ tissue, etc., or about the prospective recipient of the solid-organ tissue, e.g. sex, age, cause of death, ethnicity, medical records, medical condition, height, body mass index, etc., or the pre-transplant information additionally includes information about both donor of the solid organ tissue and about the recipient.

In a further embodiment of the present invention the pre-transplant information consists of data about the solid-organ tissue, and/or about the donor of it, can be leveraged to find an optimal recipient, e.g. by maximizing the likelihood of a high success score. For example, in the case of liver transplantation, wherein the pre-transplant information consists of FMN, FAD and NADH measurements at e.g. 30 minutes during solid-tissue hypothermic oxygenated perfusion (HOPE) at 9-11°C using Belzer MPS© UW Machine Perfusion Solution (Bridge To Life) as perfusate, and information about the donor of the liver, a success score consisting of a weighted sum of EAD as defined by Olthoff et al. (Validation of a current definition of early allograft dysfunction in liver transplant recipients and analysis of risk factors. Liver Transplant. 2010;16:943-949) and the MELD (model for end-stage liver disease) could be used in the computer model to match an optimal recipient.

Typical post-transplant parameters are frequently clinical parameters characterizing the transplant outcome, the most important ones comprise the survival of the recipient or primary non-function of the transplanted solid organ in the recipient. Other important clinical parameters comprise the concentration of lactate in the blood at different time points, INR at different time points, transcription factors, inflammation markers, tumor necrosis factors, creatinine, and others. Moreover, post-transplant parameters can also be mapped to economic values, e.g. the post-transplant costs incurred by the patient care, reimbursement, intensive care unit costs, etc.

The prediction algorithm to be used in the present invention can comprise any classification or correlation algorithm, such as a (boosted and gradient) random forest algorithm, a decision tree algorithm, a logistic regression algorithm, a neural network algorithm or a genetic algorithm. To generally train or otherwise refine the algorithm, the set of previously available pre-transplant information along with the set of the corresponding post-transplant parameter of interest of previous transplantations data are compared against the actual tissue whose prediction is sought. Using this pool of information the prediction algorithm can then learn to predict, or improve its prediction, of the parameter of interest. In this manner, the prediction algorithm can then be used to predict the post-transplant parameter. In the case of classification, the post-transplant parameter could be patient survival or solid organ graft survival after a period of time, e.g. four months, 1 year, 5 years. In this scenario, the prediction algorithm could yield a binary yes-no prediction, or it could determine the probability of a successful outcome regarding patient survival or solid organ graft survival. In the case of a regression algorithm, the post-transplant parameter could be Factor V or peak AST after a set period of time, e.g. 48 hours post-transplant or seven days post-transplant. In this scenario, the prediction algorithm yields a numeric value of the predicted post-transplant parameter. Preferably, the prediction algorithm is periodically refined as more actual data on transplant-outcomes become available to it, such as periodically training the algorithm every n additional data points, with n ranging from 1 to 10⁸.

The metric used in the learning process of the prediction algorithm can be supplied to it. If a regression algorithm is used the metric could comprise of the mean squared error, the root mean squared error, the mean absolute error, or any other user-provided function that yields a scalar output. If a classification algorithm is used these could comprise classification accuracy, sensitivity (with a user provided cutoff), specificity (with a user provided cutoff), prevalence, detection rate, detection prevalence, balanced accuracy, precision, recall, F1 score, area under the receiver operating characteristic curve (AUROC), lift curves or any other user-provided function that yields numeric output.

The prediction algorithm can be used to create a computer model reflecting the chances of a successful transplant outcome characterized by a set of post-transplant parameters. Preferably, the computer model yields the predictions of a set of a variety of clinical parameters. In such scenario, the computer model may encompass a corresponding set of prediction algorithms, each of which may have been learned on a unique pool of information of previously available pre-transplant information along with the set of the corresponding post-transplant parameter of interest of previous transplantation.

The computer model can further combine the set of predicted post-transplant parameters and map those into a success score, e.g. by weighing each parameter to emphasize certain post-transplant parameters more than others. The success score can be used to facilitate the decision process whether to transplant tissue or not.

One aspect of the present invention is that it may build databases by storing data. The databases comprise data about the donor of the solid organ tissue, about the solid organ tissue during preservation as measured by any or a combination of the methods explained above (predictive target molecules). In case the solid organ tissue was transplanted, the database contains data about the recipient and the corresponding post-transplant outcome parameters. In case the solid organ tissue was not transplanted, the appropriate reason for the negative transplant decision can be supplied to the database. Both pre-transplant information and post-transplant outcome data can be supplied to the database as soon as it becomes available but also thereafter.

The database can then be used to refine the prediction algorithm. For example, when the data of say five new transplant outcomes are fed to the database. The resulting larger body of pre-transplant information and the corresponding post-transplant parameters can be queried from the database and fed to the prediction algorithm. Letting the prediction algorithm learn on the larger body of pre-transplant information can improve the success score and lead to better predictions of post-transplant parameters of subsequent transplantations.

The database can be on a local data carrier of the analysis unit described above or the data base can be located on a remote data carrier. In the latter case, a (secure) connection to the database can be established to query from or to add data to it via a communication protocol, e.g. via a wireless technology, e.g. Bluetooth, network protocols. Optionally, communication between analysis unit and remote database carrier can be encrypted. For example, the information between analysis unit and the remote database carrier can occur via the secure shell protocol.

Embodiments of the present invention can include a visual computer interface and printable reports in a real-time (i.e. during evaluation of the solid organ tissue) or past success scores basis, monitor the statistics of the current solid organ tissue and comparing them to the past success scores. Moreover, additional measurement data or donor-specific data can optionally be indicated and visualized on the interface.

The computer model and/or the database may be implemented in hardware or software, or a combination of the two. The computer model is preferably implemented in computer programs executing on programmable computers that each includes a processor, a storage medium readable by the processor (including volatile and nonvolatile memory and/or storage elements), and suitable input and output devices. Program code is applied to data entered using an input device to perform the functions described and to generate output information. The output information is applied to one or more output devices. Moreover, each program is preferably implemented in a high level procedural or object-oriented programming language to communicate with the analysis unit and the database. If desired, the programs can alternatively be implemented in assembly or machine language. In any case, the language may be a compiled or interpreted language. Each such computer program is preferably stored on a storage medium or device (e.g. hard disk, magnetic diskette, CD-ROM) that is readable by a general or special purpose programmable computer for configuring and operating the computer when the storage medium or device is read by the computer to perform the procedures described. The system may further be implemented as a computer-readable storage medium, configured with a computer program, where the storage medium so configured causes a computer to operate in a specific and predefined manner.

The present method according to the invention combines several aspects:
- Algorithm to link the clinical markers (such as the FMN value) to patient outcome in organ transplantation, such as liver transplantation and other clinical procedures;
- a cutoff value of the marker molecule, such as FMN (8800 AU, and corresponding concentration) allows for decision for or against transplantation;
- Combination of perfusate / organ;
- Extension to other organs, tissue, damage as a whole; and
- Measurements in dialysate, data for heart and liver perfusion present.

The present method is carried out using a spectroscopic analysis unit comprising at least one spectrometer and at least one computer processor for carrying out the at least one prediction algorithm for predicting the at least one success score. The at least one spectrometer uses UV/VIS- spectroscopy and/or Fluorescence-spectroscopy. The spectroscopic analysis unit is combined with at least one perfusion machines and/or at least one perfusion loop as will described in detail below.

The present method is explained in more detail in the following with reference to the figures. It shows:
- Fig. 1: A general scheme of the spectroscopic analysis unit;
- Fig. 2: Spectroscopic Analysis Unit, describes a first embodiment for realization of spectrometric fluorescence and absorption measurements in the perfusate based on LEDs and at least one full wavelength range light source;
- Fig. 3:: Spectroscopic Analysis Unit, describes a second embodiment for realization of spectrometric fluorescence and absorption measurements in the perfusate based on full wavelength range light sources; and
- Fig. 4: a general scheme of an embodiment of the present method according to the invention.

The amount of marker molecule (predictive target molecule), in particular of FMN in the perfusate, i.e. the fluid that is used to flush and/or perfuse solid organ tissue during explantation, harvesting, during in-vivo, or during ex-vivo preservation, correlates positively with the degree of injury or damage of the solid organ, e.g. in case of livers, kidneys, hearts, lungs, pancreas, uterus, extremities, intestines and genital organs. In case of an organ flush or machine perfusion of an organ (in vivo or ex vivo), the FMN and other marker molecules of interest are actively wash out of the tissue into the perfusate. Therefore, also the information on the degree of organ damage and injury is present in the perfusate, based on the amount of such marker molecules present there (e.g. FMN and/or FAD, and/or NADH). Therefore, a high amount of FMN as marker molecule in the perfusate reveals that there is more ischemic damage compared to when the amount of FMN is low. The ischemic damage of the organ is one of the main risks for the recipient in case of transplantation. The amount of FMN as marker molecule measured in the perfusate is used as a decision criterion amongst others to decide if a donor organ should transplanted or discarded as FMN correlates with a variety of clinically important parameters and finally with the outcome of the transplantation.

Amongst others, the amount of FMN in the perfusate correlates significantly with clinical parameters in case of liver transplantation given in the following table 1:

**Table 1**

| **Parameters that correlate significantly with the amount (concentration) of FMN in perfusate in case the liver is transplanted:** |
|---|
| arterial lactate at the end of liver transplantation |
| arterial lactate at 24 hours after liver transplantation |
| arterial lactate at 48 hours after liver transplantation |
| INR (international normalized ratio) at 24 hours after liver transplantation |
| INR at 48 hours after liver transplantation |
| Factor V at 24 hours after liver transplantation |
| Factor V at 48 hours after liver transplantation |
| Peak ALT (alanine aminotransferase) during the first week after liver transplantation |
| Graft survival after liver transplantation |
| Patient survival after liver transplantation |

FMN in the perfusate (also in combination with other parameters and/or other predictive target molecules, e.g. FAD, NADH) can be used as a predictor to judge the quality of the organ prior transplantation and consecutively group the orangs according to their FMN release (FMN cutoff value(s)) at desired time points into two (low, high) to three (low, middle, high) risk groups. These risk groups characterize the risks for the recipient when receiving the organ. Moreover, the concentration of FMN in the perfusate of ex vivo perfused human organs correlates strongly with graft function, early graft loss and patient survival. So, there is clearly a predictive value of machine perfusate analysis (respectively e.g. FMN concentration in the perfusate and/or FAD concentration and/or NADH concentration) with respect to such predictive target molecules on transplantation outcome. It is expected that the FMN is released from the mitochondria of the cells linked to the level of mitochondrial complex I injury. For donated organs (e.g. livers) that are per se marginal (DCD (donation after cardiac death) or extended criteria DBD (donation after brain death)), this evaluation technology enables the save use of such organs and therefore increasing the donor pool to save patients on the waiting list. Also for seemingly perfect organs, this evaluation step is useful, because it implements an additional safety feature to check the organ quality prior transplantation.

Moreover, apart from FMN as a predictive target molecule to quantify organ damage/injury and thus predict the success rate of the transplantation, there are also other molecules that have proven their predictive value. Other predictive target molecules (predictive parameters) for tissue/organ (ischemic) damage/injury in the perfusate are flavin adenine dinucleotide (FAD), nicotinamide adenine dinucleotide (NAD) and lactate. Moreover ALT, glucose and AST in perfusate can also be considered. The amount of these predictive target molecules (substances) in the perfusate (e.g. during machine perfusion) also strongly correlate with the organ damage/injury, respectively with the quality of the organ and finally with the outcome of the transplantation. For example high values of FMN (e.g. >9000 AU) in combination with high amounts of lactate (e.g. >4mmol/l) clearly indicate an organ of poor quality.

The amount of these predictive target molecules in the perfusate can be measured at desired time points by taking perfusate samples out of the perfusion loop. On the other hand, in situ (on line) and continuous measurements are possible during organ machine perfusion. Moreover, these predictive target molecules can also be measured in the perfusate after an organ flush was executed e.g. on the back table or even in the preservation solution where the organ is stored during static cold storage.

The concentration of e.g. FAD, FMN, NADH, AST, ALT, glucose, lactate and so on (predictive target molecules) in the perfusate can be measured at discrete time points with standard standalone laboratory measurement devices/methods (e.g. Radiometer ABL90, Abaxis Piccolo Xpress, UV/VIS spectroscopy, MRI analysis, chromatography) or continuously and in situ (on-line) in the perfusate during machine perfusion. Online measurements of glucose and lactate can be done continuously and in-situ with these two systems (CITSens Bio and MeMo, C-CIT Sensor AG).

Measurements of FMN, FAD and NADH in the perfusate in a continuous or non-continuous manner are done by the spectroscopic analysis unit by detecting fluorescence and absorption spectra. The non-continuous FMN and FAD measurement is done by sampling the perfusate into e.g. a cuvette or an Eppendorf tube. This sample (e.g. cuvette or Eppendorf tube) is then put into the sample holder of the spectroscopic analysis unity for spectrometric analysis. On the other hand, the continuous measurement during organ perfusion is done by means of a flow cell (flow-through cell, flow-through cuvette) integrated into the spectroscopic analysis unit. Within such a spectrometric flow cell, perfusate from the machine perfusion loop is permanently flowing through this flow cell (senor) for spectroscopic analysis to get real time data and time resolved data. Therefore, the spectroscopic analysis unity respectively the flow cell needs to be connected via tubes and e.g. LuerLock connectors to the perfusion loop assembly of the perfusion machine. After passing through the flow cell, the perfusate can be given back to the machine perfusion loop or can be discarded. Permanent and stable flow has to be established through the flow cell to get reliable measurements. This can be realized by means of a suitable pressure difference between inlet and outlet of the flow cell given by the different pressure levels in the perfusion loop. Alternatively, a pump (e.g. roller pump) can be used to pump the fluid through the flow cell.

Furthermore, the spectroscopic analysis unit is able to measure directly through a special tube of the perfusion loop itself. In this case, no sample holder or flow cell in needed, because the spectroscopic analysis unit respective the senor unit is directly connected to a desired outer tube of the perfusion loop of the perfusion machine. A possible solution in this case is a clamp on or thread through spectroscopic sensor unit.

In an embodiment where the perfusate is sanguineous (e.g. whole blood, or perfusate with red blood cells), the perfusate can be directly analyzed in the spectroscopic analysis unit. Alternatively, a portion of the perfusate passes a hemodialysis filter separating (blood) plasma from blood cells (e.g. red blood cells) first and the plasma portion then flows through the flow cell. Afterwards, the blood plasma can or cannot be recirculated to the perfusion loop. Alternatively, the continuous separation of blood into blood cells and blood plasma can also be realized with microfluidic devices (micro reactors). In case of sample-based analysis, the (bood) plasma is spectrometric analyzed after filtration or centrifugation of blood cells.

Conductivity and capacitance measurements of the perfusate / body fluid can be additionally implemented in the spectroscopic analysis unit. Conductivity and/or capacitance measurements of the perfusate / body fluid can also help to quantify organ quality or detect diseases in patients.

Generally, the spectroscopic analysis unit can be integrated into standalone in vitro diagnostic device where taken perfusate samples (e.g. in cuvette or Eppendorf tube) are spectroscopically analyzed (e.g. by absorption and fluoresce) to detect the molecules of interest (predictive target molecules) e.g. FMN. Moreover, apparat from perfusate, several body fluids can be spectroscopically analyzed to measure the amount of specific molecules to detect/diagnose diseases, dysfunctions, genetic defects, cancer and infections of the patient.

On the other hand, the spectroscopic analysis unit can be fully integrated (being part of it) into all state of the art machine perfusion technologies for several organs, types of perfusate and in all temperature ranges (hypotherm, subnormothermic and normothermic). In this case, e.g. FMN can be directly measured (real time and continuous) in the perfusion loop by a flow cell (e.g. a disposable one) or directly through the tube of the perfusion loop itself.

Moreover, the spectroscopic analysis unit can be used as an add-on product to all state of the art organ perfusion technologies. Therefore, the spectroscopic analysis unit has to be connected to the desired organ perfusion loop via tubes and e.g. LuerLock connectors. After passing through the flow cell (of the spectroscopic analysis unit), the perfusate can be given back to the machine perfusion loop or can be discarded.

Furthermore, the spectroscopic analysis unit can be used as an add-on product to all state of the art dialysis machines. The used dialysate leaving the dialysis filter is fed to the flow cell of the spectroscopic analysis unit for spectrometric analysis to detect the amount of molecules of interest (predictive target molecules) to detect/diagnose diseases, dysfunctions, genetic defects, cancer and infections of the patient. Generally, the spectroscopic analysis unit can also be fully integrated into all state of the art dialysis machines.

Generally, flavins (e.g. FMN & FAD as predictive target molecules), which have two absorption maxima at ∼ 360-390 nm and at ∼ 440-470 nm. The two most common biological forms of Flavin are flavin mononucleotide (FMN, riboflavin 5' phosphate) and flavin adenine dinucleotide (FAD), where both are fluorescent. Free FMN absorbs maximally at 373 nm and 445 nm (ε = 10,400 and 12,500 M⁻¹ cm⁻¹, respectively) whereas free FAD has maxima at 375 nm and 450 nm (ε = 9300and 11300 M⁻¹ cm⁻¹, respectively). The fluorescence emission maxima for FAD and FMN is at 525 nm.

The applied spectrometer(s) of spectroscopic analysis unit is typically operating in the UV/VIS range but can also cover a broader range (100-3000nm). Said spectroscopic analysis unit is used preferably for measuring the fluorescence of Flavin Mononucleotide (FMN) and/or FAD as a fragment of mitochondrial complex I. Detecting FMN and/or FAD in the perfusate during e.g. ex-vivo machine perfusion allows detecting the extent of (ischemic mitochondrial) damage/injury to solid organ grafts and tissue prior to transplantation - in particular this method is applicable to all solid organs and tissue in an ex vivo perfusion system. The FMN and/or FAD signal intensity (linked to the concentration in perfusate) as extracted by the fluorescence spectra can be used to evaluate the degree of (reperfusion) injury/damage of the organ. The real-time or non -real-time measurement of FMN and/or FAD in the perfusate facilitates clinical decision making (whether an organ should be transplanted or not, minimizing the transplantation risks for the recipient) by optimizing the matching process of the graft and the recipient and hereby improving the survival and quality of life of the recipient after transplantation. In addition to liver transplantation, this method is applicable to any ex vivo perfused tissue, preferably to solid organ tissue such as liver, heart, lungs, kidney, pancreas, uterus, extremities, genital organs or intestine.

FMN is detected by fluorescence spectroscopy. In detail, a least one light probe is connected to at least one light source (LED and/or Full range (100-3000nm, e.g. Halogen) combined with bandpass filter) to emit almost monochrome light at a wavelength of around 445 nm (and/or around 373 nm) for excitation on the perfusate flow or perfusate sample. At least one receiver probe connected to at least one spectrometer (placed preferably 90° to the light probe) with sufficiently high resolution (e.g. 4.6 nm) and sensitivity was used to quantify the proportion of emitted fluorescent light by the FMN molecule. The fluorescence emission maximum of FMN was measured between 475-600nm, more precisely at 525nm.

FAD is detected by fluorescence spectroscopy. In detail, at least one light probe was connected to at least one light source (LED and/or Full range (100-3000nm, e.g. Halogen) combined with bandpass filter) to emit almost monochrome light at a wavelength of around 450 nm (and/or around 375 nm) for excitation on the perfusate flow or perfusate sample. At least one receiver probe connected to at least one spectrometer (placed preferably 90° to the light probe) with sufficiently high resolution (e.g. 4.6 nm) and sensitivity was used to quantify the proportion of emitted fluorescent light by the FAD molecule. The fluorescence emission maximum of FAD was measured between 475-600nm, more precisely at 525nm.

Nicotinamide adenine dinucleotide (NAD) exists in two forms: an oxidized and reduced form, abbreviated as NAD+ and NADH respectively. Similar to FMN, also the amount of NADH in the perfusate quantifies the (ischemic) injury/damage and the mitochondrial function of the organ respectively tissue. Therefore, also NADH (respectively NAD+, NAD) is a predictive target molecule to guess the success rate respectively the outcome of the transplantation. NAD, NAD+, NADH are detected by fluorescence spectroscopy in the perfusate. NADH absorbs light at 320-380nm (maxima at 340nm) and emits fluorescent light in the 420-480nm range (maxima at 463nm).

By means of the spectroscopic analysis unit, also the UV/VIS absorbance spectroscopy technology can be implemented in the perfusion loop to undertake light absorbance measurements of the perfusate in order to quantify the amount of molecules of interest (predictive target molecules) to e.g. the quality of the organ and therefore predict the outcome of the transplantation. For absorbance measurements, light probe and receiver probe are aligned with each other.

Further methods that can be applied to detect the predictive target molecules in the perfusate and/or the body fluids are UV-VIS-spectroscopy, fluorescence spectroscopy, Raman spectroscopy, mass spectroscopy, circular dichroism spectroscopy, (near) infrared spectroscopy.

Generally, the spectroscopic analysis unit is used to detect the amount of predictive target molecules (e.g. FMN and/or FAD) in the perfusate via florescence and/or absorption spectroscopy. Said spectroscopic analysis unit can measure only fluorescence spectra (fluorescence spectroscopy), or only absorption spectra (absorption spectroscopy) or applied both methods.

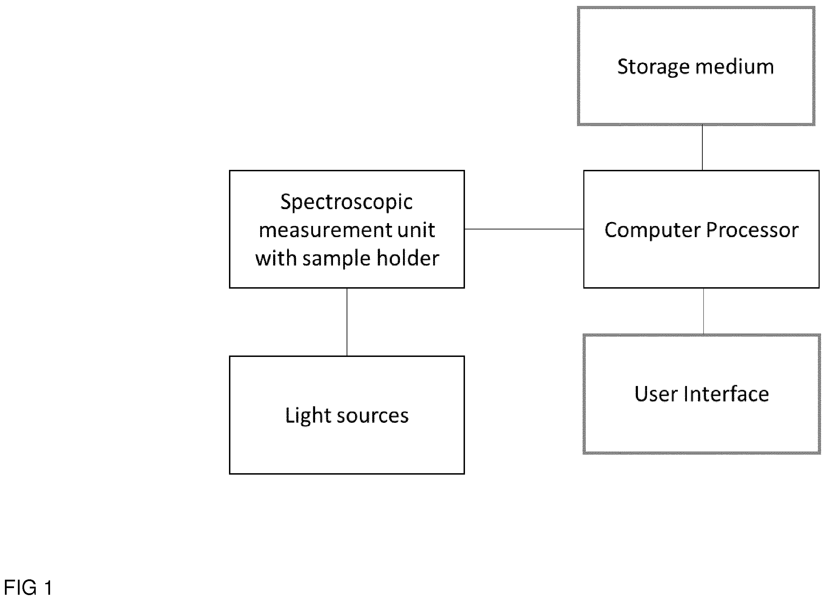

Part of the spectroscopic analysis unit are light sources, a spectroscopic measurement unit with sample holder, a computer processor connected to a storage medium and a user interface (see Fig. 1). Further parts of the spectroscopic analysis unit are a data acquisition, processing and storage unit as well as a controlling unit that switches, synchronizes and controls light sources, bandpass filters, optical filters, shutters and spectrometers. Moreover, several state of the art data and connection interfaces are part of the system.

Furthermore, the spectroscopic analysis unit can be combined with all state of the art perfusion machines. Therefore, the sensor unit is able to measure directly spectroscopically (fluorescence and/or absorption spectroscopy) through a (special) tube of the perfusion loop itself of the perfusion machine. In this case, no sample holder or flow cell in needed, because the spectroscopic analysis unit is directly connected to a desired outer tube of the perfusion loop of the perfusion machine. A possible solution in this case is a clamp on or thread through spectroscopic sensor unit. Alternatively, the sensor unit is connected to a special flow though device that is part of the perfusion loop. This special flow though device is preferably disposable and build in by the manufacturer of the tube set for the desired perfusion machine.

Several spectrometers with variable wavelength operating ranges (e.g. 200-3000nm), different resolution and sensitivity can be part of the spectroscopic analysis unit in order to detect and screen different predictive target molecules with florescence and/or absorption spectroscopy.

Generally, all light sources and be operated in a pulsed and non-pulsed (continuous) manner. For absorption spectroscopy (e.g. light probe 3), generally a full wavelength range (e.g. 200-3000nm) light source is used. Suitable here is for example a white light LED, Halogen lamp, Deuterium Tungsten Halogen lamp, Xenon lamp and so on.

In case of fluorescence spectroscopy, the perfusate is excited with (nearly) monochrome light and/or narrow wavelength bands (depending on the type of light source) at a wavelength where the desired predictive target molecule has an absorption maximum. The response of such an excitation is then detected with the spectrometer as fluorescence light at the characteristic emission maxima of the desired predictive target molecule. For fluorescence spectroscopy, different LEDs at different wavelength can be used and connected to the desired light probes (e.g. light probe 1 and 2) to excite the perfusate at different wavelengths to enable a screening for different fluorescenting predictive target molecules.

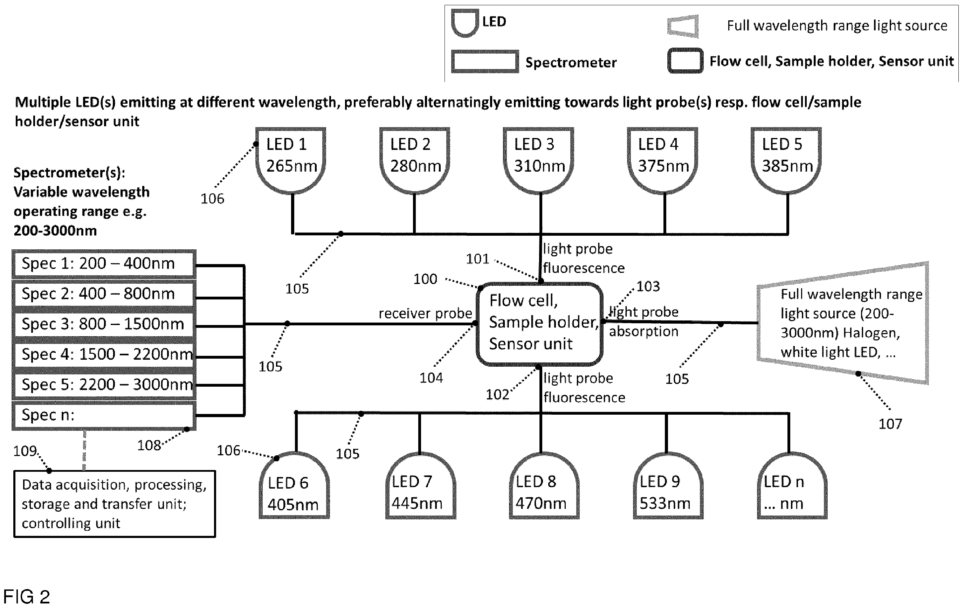

Fig. 2 represents an embodiment of the spectroscopic analysis unit to realize fluorescence and/or absorption measurements in the perfusate. In a preferred embodiment, the spectroscopic analysis unit comprises at least one LED light source (106) emitting nearly monochromatic light at the desired wavelength for excitation of the perfusate to at least one light probe fluorescence (e.g. 101 or 102). This light probe(s) (101, 102) is part of the Flow cell/Sample holder/Sensor unit (100). Moreover, a full wavelength range light source (107) emitting light of full wavelength range is connected to the Flow cell/Sample holder/Sensor unit (100) via the light probe absorption (103). In case of fluorescence spectroscopy, the light probe (101, 102) is positioned at 90° to the receiver probe (104). For absorption spectroscopy measurements, the light probe (103) is aligned (at 180°) to the receiver probe (104). The receiver probe (104) is part of the Flow cell/Sample holder/Sensor unit (100). The different possible LEDs (106) are emitting at different wavelength, preferably alternatingly only one LED is emitting towards one light probe (101, 102). At the same time, the receiver probe (104) is detecting the fluorescence light emissions of the perfusate and send this light information via optical fiber cables (105) to the spectrometer(s) (108) for detection and analysis. Fluorescence and absorption measurements of the perfusate are preferably alternatingly executed and the spectra is always detected via the receiver probe (104). When absorption measurements are executed, the full wavelength range light source (107) is sending light via the optical fiber cable (105) to the light probe absorption (103). All detected spectra are handled by the Data-Acquisition/Processing/Storage/Transfer-Unit (109). The controlling of light sources (106, 107), shutters and spectrometer(s) (108) is realized via the controlling unit (109).

Apart from LEDs, also full wavelength range light sources (e.g. 200-3000nm) can be applied for fluorescence spectroscopy. Therefore, the light beam emitted e.g. by a halogen lamp hits a switchable and controllable bundle of optical filters (e.g. bandpass filters) to excite the perfusate in the desired narrow wavelength band (e.g. 440-460nm) that passes the bandpass filters. The different bandpass filters can be individually controlled to enable the excitation in the desired wavelength range. In a preferred embodiment, every individual single and round filter disk has an empty position, where no filtration occurs. On every filter disk, there can be several individual bandpass filters. By turning the filter disk around the central axis, the wavelength that passes through the filter(s) and excites the perfusate can be changed. Every switchable and controllable bandpass filter bundle can consist of several individual filter disks to enable fluorescence screening over a broad wavelength range. In addition, filter disks that only reduce the light intensity without any filtration can also be part of the system.

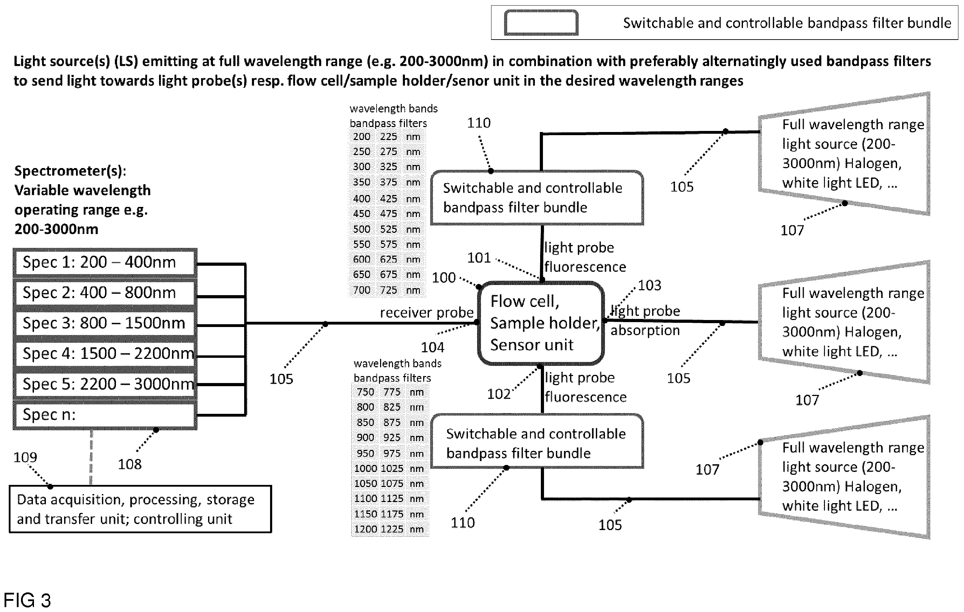

Fig 3 represents a spectroscopic analysis unit using a full wavelength range light source (107) which emits light that passes a switchable and controllable bandpass filter bundle (110). The different bandpass filters inside the bundle can be individually controlled to enable the excitation in the desired wavelength range.

Aside from the release of FMN, FAD and NADH into the perfusate, a variety of other predictive target molecules are released and can be detected, namely Xanthine, Hypoxanthine, Succinate, Xanthosine, Nicotinic Acid, Nicotinamide Adenine Dinucleotide (NAD/NADH), Flavin Adenine Dinucleotide (FAD/FADH), Inosine, Inosine-5'-monophosphate, 8-hydroxyguanosine, Uric acid, Biliverdin, Protoporphyrin, Purine, Riboflavines, Uracil, Uridine, 8-Hydroxyguanosine, adenosine tri-phosphate, adenosine-di-phosphate, Malonate, Pyruvate, Aconitate, Fumarate, Malate, Aspartate, Citrate, Aconitate, Adenine, Propionylcarnitine, Choline, Lactate, Proline, Leucine, Tryptophan, Phenylalanine, Tetramethylrhodamine, Adenosindiphosphat (ADP), Adenosintriphosphat (ATP), Creatine, N-Acetyl-L-glutamic acid in the perfusate. These molecules are measured and monitored by spectroscopic methods, e.g. nuclear magnetic resonance spectroscopy analysis of the perfusate at different time points during perfusion. This data, together with the FMN measurement and functional tests (bile production, coagulation), will further improve the assessment of the organ/tissue (e.g. liver) graft hereby improving post-transplant survival of the recipients.

The combination of available information in any of the embodiments described above can be used in a prediction algorithm.

For example, in the case of liver transplantation, wherein the amount of FMN is measured during hypothermic oxygenated perfusion (HOPE), wherein the perfusion is operated at a temperature of 4-12°C, preferably at 9-11°C, using Belzer MPS UW Machine Perfusion Solution (Bridge To Life). The peak value of FMN e.g. at 30 minutes of perfusion is used as pre-transplant information. Using the AUROC metric, the computer model for the success criterion "early allograft dysfunction (EAD) as defined in Olthoff et al." in this simple example consists of a decision rule wherein EAD is predicted when the amount of FMN is higher than a threshold (e.g. 8800 arbitrary units, which can be mapped to a concentration amount of FMN in weight per volume of fluid, e.g. micro grams per milliliter, by a device specific calibration curve), and wherein EAD is not expected when the amount of FMN is lower than said threshold. In this context, the prediction algorithm can facilitate the decision whether to transplant the liver or to discard it.

As a second example, the success score comprises the combination of the INR at 24h, the peak AST of the recipient at 7 days post-transplant and the number of days that the recipient has to spend on the ICU, weighing the first two parameters more heavily than the latter. In such a way, the economic contribution (represented through the ICU stay) is weighted less than the contribution characterizing the health condition of the recipient.

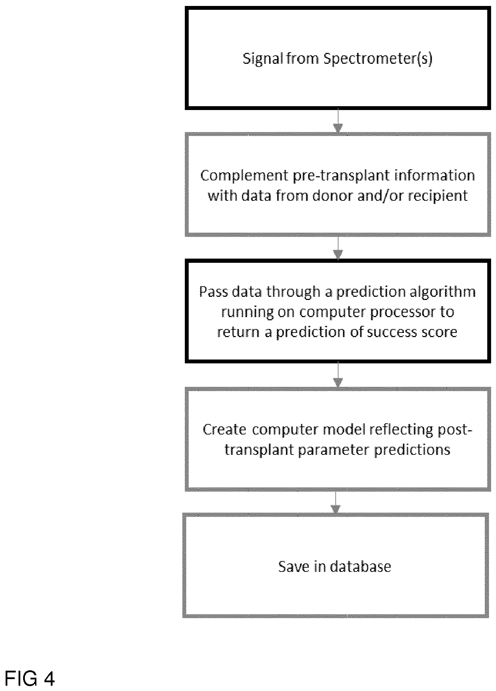

The scheme of Figure 4 summarizes the steps taken by the present method: The spectrometer provides a signal corresponding to concentration of a marker molecule as one pre-transplant information. This pre-transplant information is complemented with data from donor and/or recipient. The (combined) data are passed through a prediction algorithm running on the computer processor to return a prediction of success score. A computer model is created which reflects the post-transplant parameter predictions. The computer model is saved in a database.

### Definitions, technical terms:

**Perfusate:** Is the fluid that is used to flush and/or perfuse solid organ tissue in-vivo or ex-vivo. Also the solution that is applied to human organs for preservation purposes during static cold storage on ice is entitled perfusate in this context. Moreover, also body fluid and perfusate have the same meaning in the framework of this patent. Perfusate can be whole blood, sanguineous or asanguineous perfusate, a physiological saline solution, a perfusate based on an artificial oxygen carrier, modified blood (e.g. leukocyte depleted) or a tailor-made solution like: University of Wisconsin machine perfusion solution (Belzer-MPS®); Institute-George-Lopez-1 (IGL-1) solution; Ringerfundin, B. Braun; Plasma-Lyte A, Baxter)
**body fluid:** blood, blood plasma, urine, bile, spittle, sudor, lymphe, gastric juice, secretion of pancreas, breast milk, vaginal secretion, tear fluid, nasal discharge, sperm, menstrual fluid, surfactant
**marker molecule(s) or predictive target molecule(s):** Are molecules/substances that are measured real time (online, continuous) or non-real time (sample based) in the perfusate. The amount (concentration) of these predictive target molecules present in the perfusate quantifies the (ischemic) injury/damage of human organs/tissue and therefore predict the success rate and the outcome of the transplantation. Predictive target molecules/marker molecules are FMN, FAD, NADH, lactate and others.
**Reperfusion injury:** Reperfusion injury is the tissue damage caused when blood supply returns to tissue after a period of ischemia or lack of oxygen (anoxia or hypoxia).
**Ischemia:** Ischemia, ischemic (or ischaemia) is a restriction in blood supply to tissue, causing a shortage of oxygen, nutrients and glucose needed for cellular metabolism to keep tissue alive.
**Ex vivo:** outside of the body;
**In vivo:** inside the body
**Perfusion loop:** The perfusion loop should be connected to the human organ by suitable means such as cannulas. Moreover, the perfusion loop is connected to the durable of the perfusion machine. The perfusion loop is technically realized via the tube set.
**Tube set:** The tube set is the technical realization of the perfusion loop to enable the desired organ/tissue machine perfusion that includes all tubes, connectors, sensors, valves, ports, oxygenators, filters, dialyzers, pump heads, clamps and so on.

### Abbreviations:

ADP: Adenosindiphosphat
Al: Artificial intelligence
ALT: Alanine Aminotransferase
AST: Aspartate-Aminotransferase
ATP: Adenosintriphosphat
AUROC: area under the receiver operating characteristic curve
DBD: donation after brain death
DCD: donation after circulatory death
D-HOPE: Dual Hypothermic Oxygenated Maschine Perfusion
EAD: early allograft dysfunction
FAD: Flavin Adenine Dinucleotide
FMN: Flavin Mononucleotide
HOPE: Hypothermic Oxygenated Machine Perfusion (HOPE)
INR: international normalized ratio
LED: light-emitting diode
LT: liver transplantation
MELD: model for end-stage liver disease
NAD, NADH: Nicotinamide adenine dinucleotide
NRP: Normothermic regional perfusion
ROS: reactive oxygen species

### List of Reference Signs

- 100: Flow cell, Sample holder, Sensor unit
- 101: light probe fluorescence
- 102: light probe fluorescence
- 103: light probe absorption
- 104: receiver probe
- 105: optical fiber cable
- 106: LED
- 107: full wavelength range light source
- 108: spectrometer
- 109: Data acquisition, processing, storage and transfer unit; controlling unit
- 110: Switchable and controllable bandpass filter bundle

## Claims

1. Method for evaluating damage of an organ tissue, in particular ischemic damage/injury of an organ tissue,
**characterized by**
- measuring the concentration of at least one marker molecule in the perfusate of the organ tissue,
- wherein the measured concentration of the at least one marker molecule in the perfusate is used in at least one computer based prediction algorithm for generating at least one success score,
- wherein the success score has been previously defined based on at least one parameter value of at least one pre-defined parameter;
- wherein the at least one parameter value is determined after a transplantation of the organ tissue; and
- wherein based on the at least one success score at least one signal and/or at least one set of data is generated for facilitating the decision, if the organ tissue is suitable for transplantation or not.

2. Method according to claim 1, wherein the success score reflects the degree of organ tissue damage.

3. Method according to claim 1, **characterized in that** machine learning and artificial intelligence is applied for characterizing the condition of the organ tissue.

4. Method according to one of the preceding claims, **characterized in that** the at least one success core corresponds to the concentration of the at least one marker molecule in the perfusate, wherein a previously determined threshold value of the at least one marker molecule is used for generating the at least one signal and/or at least one set of data for facilitating the decision of an organ issue transplantation prior to the organ tissue transplantation.

5. Method according to one of the preceding claims, **characterized in that** the computer based prediction algorithm is a regression algorithm or classification algorithm, wherein the pre-defined parameters used by the least one prediction algorithm are pre-transplanted information and post-transplant parameters.

6. Method according to one of the preceding claims, **characterized in that** the computer model encompass a corresponding set of prediction algorithms, each of which may have been learned on a unique pool of information of previously available pre-transplant information along with a set of the corresponding post-transplant parameter of interest of previous transplantation.

7. Method according to one of the preceding claims, **characterized in that** the computer model further combine the set of predicted post-transplant parameters and map those into a success score, preferably by weighing each parameter to emphasize certain post-transplant parameters more than others, and said success score is used to facilitate the decision process whether to transplant tissue or not.

8. Method according to one of the preceding claims, **characterized in that** the prediction algorithm uses information stored in a database, wherein the database comprises pre-transplant parameters and post-transplant parameters.

9. Method according to one of the preceding claims, **characterized in that** the at least one marker molecule is selected from a group comprising FMN, lactate, FAD, NADH, Alanine Aminotransferase (ALT), Aspartate Aminotransferase (AST), Glucose, wherein FMN and FAD are the most preferred marker molecules.

10. Method according to one of the preceding claims, **characterized in that** the concentration of the at least one marker molecule in the perfusate is measured in real time (online, continuous) or non-real time (sample based).

11. Method according to one of the preceding claims, **characterized in that** the pre-transplant parameters comprise in addition to the concentration of the at least one marker molecule information about the donor of the solid-organ tissue, in particular sex, age, cause of death, ethnicity, medical records, medical condition, height, body mass index, ischemia time of the solid organ tissue; and / or about the prospective recipient of the solid-organ tissue, in particular sex, age, cause of death, ethnicity, medical records, medical condition, height, body mass index,

12. Method according to one of the preceding claims, **characterized in that** the post-transplant parameters comprise the survival of the recipient or primary non-function of the transplanted solid organ in the recipient, the concentration of lactate in the blood at different time points, INR at different time points, transcription factors, inflammation markers, tumor necrosis factors, creatinine.

13. A spectroscopic analysis unit used in a method according to one of the preceding claims comprising at least one spectrometer and at least one computer processor for carrying out the at least one prediction algorithm for predicting the at least one success score.

14. Spectroscopic analysis unit according to claim 13, **characterized in that** the at least one spectrometer uses UV/VIS- absorbance spectroscopy or Fluorescence-spectroscopy.

15. Spectroscopic analysis unit according to claim 13 and 14, **characterized in that** the unit is combined with at least one perfusion machines and/or at least one perfusion loop.
